(19) Europäisches Patentamt
European Patent Office
Office européen des brevets

(11) Numéro de publication: **0 120 741**
**B1**

# (12) FASCICULE DE BREVET EUROPEEN

(45) Date de publication du fascicule du brevet: **08.10.86**

(51) Int. Cl.⁴: **A 61 K 31/385**

(21) Numéro de dépôt: **84400366.5**

(22) Date de dépôt: **23.02.84**

(54) **Nouveaux médicaments dérivés de la chloro-4 dithiole-1,2 one-3 et les compositions médicinales qui les contiennent.**

(30) Priorité: **24.02.83 FR 8303003**

(43) Date de publication de la demande: **03.10.84 Bulletin 84/40**

(45) Mention de la délivrance du brevet: **08.10.86 Bulletin 86/41**

(84) Etats contractants désignés: **BE CH DE FR GB IT LI LU NL SE**

(56) Documents cités:
**DE - B - 1 278 701**
**FR - A - 1 498 374**

(73) Titulaire: **RHONE-POULENC SANTE, Les Miroirs 18 Avenue d'Alsace, F-92400 Courbevoie Cedex (FR)**

(72) Inventeur: **Bourzat, Jean-Dominique, 35, Rue Santos-Dumont, F-75015 Paris (FR)**
Inventeur: **Cotrel, Claude, 17 A avenue du Dr. Arnold Netter, F-75012 Paris (FR)**
Inventeur: **Farge, Daniel, 30, rue des Pins Sylvestres, F-94320 Thiais (FR)**
Inventeur: **Paris, Jean-Marc, 8, rue des Acacias, F-77360 Vaires S/Marne (FR)**
Inventeur: **Taurand, Gérard, 13, Passage Saillenfait, F-94000 Creteil (FR)**

(74) Mandataire: **Le Goff, Yves et al, RHONE-POULENC RECHERCHES Brevets Pharma 25, Quai Paul Doumer, F-92408 Courbevoie (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

La présente invention concerne, à titre de médicament, les dérivés de la chloro-4 dithiole-1,2 one-3 de formule générale:

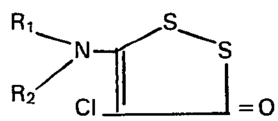

(I)

dans laquelle

— soit $R_1$ représente un atome d'hydrogène ou un radical alcoyle. et $R_2$ représente un radical phényle non substitué ou substitué par un ou plusieurs substituants choisis parmi les atomes d'halogène et les radicaux alcoyle contenant 1 à 5 atomes de carbone en chaîne droite au ramifiée, hydroxy, amino, alcoylamino, dialcoylamino, alcoyloxy, alcoylthio, alcoylsulfinyle, alcanoyle, alcoyloxycarbonyle, carbamoyle, sulfamoyle, cyano, nitro, trifluorométhyle et phényle,

— soit $R_1$ et $R_2$ forment ensemble avec l'atome d'azote auquel ils sont liés un hétérocycle à 5 ou 6 chaînons pouvant contenir éventuellement un second hétéroatome choisi parmi l'oxygène et l'azote éventuellement substitué par un radical alcoyle, étant entendu que les radicaux et portions alcolye cités ci-dessus ou qui seront cités par la suite contiennent, sauf mention spéciale, 1 à 4 atomes de carbone et sont en chaîne droite ou ramifiée, ainsi que leurs sels d'addition avec les acides lorsqu'ils existent.

Les produits de formule générale (I) sont déjà connus par F. BOBERG, Annalen der Chemie 681, 169-177 (1965) ainsi que par les brevets allemands 1 242 324 et 1 278 701 et le certificat d'addition n° 94 485 au brevet français 1 498 374. Pour autant que les composés de formule générale (I) ne sont pas expressément décrits dans les documents ci-dessus, ils peuvent être préparés par action d'une amine de formule générale:

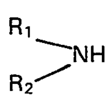

(II)

sur la dichloro-4,5 dithiole-1,2 one-3 de formule:

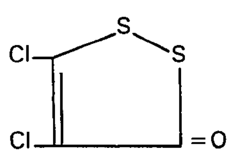

(III)

On opère généralement dans un solvant organique tel qu'un alcool comme le méthanol, ou le diméthylformamide, en présence d'un accepteur d'acide tel qu'un carbonate ou bicarbonate alcalin comme le carbonate ou le bicarbonate de potassium, ou encore une base organique comme le nitrilo-tris(propanol-2) à une température comprise entre 0 et 80°C.

Les produits de formule générale (I) dans laquelle $R_1$ représente un radical alcoyle et $R_2$ est défini comme précédemment peuvent également être préparés par action d'un agent d'alcoylation de formule générale:

$$R'_1X \qquad (IV)$$

dans laquelle $R'_1$ représente un radical alcoyle contenant 1 à 5 atomes de carbone en chaîne droite ou ramifiée et X représente un atome d'halogène ou un reste d'ester réactif tel que mésyloxy ou tosyloxy, sur un produit de formule générale (I) dans laquelle $R_1$ représente un atome d'hydrogène et $R_2$ est défini comme précédemment, c'est-à-dire un produit de formule générale:

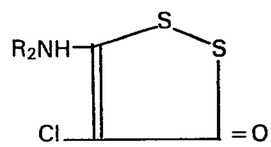

(V)

La réaction s'effectue par toute méthode connue de l'homme du métier pour effectuer une N-alcoylation d'amine sans toucher au reste de la molécule. Il est généralement avantageux d'opérer en présence d'un agent de condensation tel que l'hydrure de sodium et d'effectuer la condensation dans le diméthylformamide.

La dichloro-4,5 dithiole-1,2 one-3 de formule (III) peut être préparée selon la méthode décrite par F. BOBERG, Ann. Chem. 681, 169 (1965).

Comme il a été dit précédemment, les produits de formule générale (I) sont connus par la publication de BOBERG (déjà cité), par les brevets allemands 1 242 324 et 1 278 701 et par le certificat d'addition n° 94 485 au brevet français 1 498 374. Le premier document ne donne aucune application pour les produits décrits, et les trois brevets mentionnés enseignent que les produits revendiqués possèdent des propriétés pesticides, fongicides et antimicrobiens agricoles c'est-à-dire des propriétés rendant les produits utilisables dans le domaine phytosanitaire.

Il a maintenant été trouvé, et c'est ce qui fait la caractéristique principale de l'invention, que les produits de formule générale (I) ainsi que leurs sels, lorsqu'ils existent, présentent des propriétés pharmacologiques intéressantes susceptibles de les rendre utiles dans le traitement de fond de la maladie rhumatismale.

Ainsi les produits de formule générale (I) se sont montrés actifs à des concentrations de $10^{-5}$ à $10^{-6}$M dans le test de J. SCHNYDER et M. BAGGIOLINI (J. Exp. Med. 148, 1449, 1978) permettant de mesurer l'effet de produits sur l'activation des macrophages in vitro.

En outre, les produits de formule générale (I) présentent une toxicité faible. Leur $DL_{50}$ est généralement supérieure à 900 mg/kg par voie orale chez la souris en administration unique.

Pour l'emploi médicinal, il peut être fait usage des produits de formule générale (I) tels quels ou, lorsqu'ils existent, à l'état de sels pharmaceutiquement acceptables, c'est-à-dire non toxiques aux doses d'utilisation.

Comme sels pharmaceutiquement acceptables

peuvent être cités par exemple les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophyllineacétates, salicylates, phénolphtalinates, méthylène-bis-β-oxynaphtoates ou des dérivés de ces composés.

Sont particulièrement intéressants les produits de formule générale (I) dans laquelle soit $R_1$ représente un atome d'hydrogène et $R_2$ représente un radical phényle non subsitué ou substitué par un ou plusieurs substituants choisis parmi les atomes d'halogène et les radicaux alxoyle contenant 1 à 4 atomes de carbone (en chaîne droite ou ramifiée), amino et phényle, soit $R_1$ et $R_2$ forment ensemble avec l'atome d'azote auquel ils sont liés un cycle pipéridine ou morpholine.

Les exemples suivants montrent comment peuvent être préparés les produits servant de matière active pour la préparation des médicaments selon l'invention.

*Exemple 1*

A une suspension de 11 g d'hydrogénocarbonate de potassium dans 150 $cm^3$ de méthanol contenant 14,9 g d'isopropyl-4 aniline, on ajoute 18,7 g de dichloro-4,5 dithiole-1,2 one-3. Le mélange réactionnel est agité pendant 20 heures à une température voisine de 20°C. On ajoute ensuite 750 $cm^3$ d'eau distillée et l'émulsion obtenue est extraite par 2 fois 500 $cm^3$ au total de chlorure de méthylène. Les phases organiques sont réunies et séchées sur du sulfate de magnésium, filtrées puis concentrées à sec sous pression réduite (2,7 Pa) à 40°C. Après recristallisation dans un mélange d'acétonitrile et d'éther isopropylique (9/1 en volume), on obtient 20 g de chloro-4 (isopropyl-4 phénylamino)-5 dithiole-1,2 one-3 sous forme de cristaux jaunes fondant à 114°C.

La dichloro-4,5 dithiole-1,2 one-3 peut être préparée selon F. BOBERG, Ann. Chem. 681, 169 (1965).

En opérant de la même manière mais à partir des amines correspondantes, on peut préparer les produits suivants:

*Exemple 2*

Chloro-4 (isopropyl-2 phénylamino)-5 dithiole-1,2 one-3 sous forme d'une poudre jaune fondant à 136°C.

*Exemple 3*

Chloro-4 [(éthyl-1 propyl)-4 phénylamino]-5 dithiole-1,2 one-3 sous forme d'une poudre beige fondant à 135°C.

*Exemple 4*

Chloro-4 phénylamino-5 dithiole-1,2 one-3 sous forme d'une poudre jaune clair fondant à 164°C.

*Exemple 5*

Chloro-4 (méthyl-4 phénylamino)-5 dithiole-1,2 one-3 sous forme d'une poudre jaune clair fondant à 174°C.

*Exemple 6*

Chloro-4 (t-butyl-4 phénylamino)-5 dithiole-1,2 one-3 sous forme d'une poudre jaune fondant à 134°C.

*Exemple 7*

Chloro-4 (hydroxy-4 phényl)-5 dithiole-1,2 one-3 sous forme d'une poudre jaune clair fondant à 215°C.

*Exemple 8*

Chloro-4 (hydroxy-3 phénylamino)-5 dithiole-1,2 one-3 sous forme d'une poudre brune fondant à 134 à 135°C.

*Exemple 9*

Chloro-4 (fluoro-4 phénylamino)-5 dithiole-1,2 one-3 sous forme d'une poudre jaune fondant à 193°C.

*Exemple 10*

Chloro-4 (dichloro-3,4 phénylamino)-5 dithiole-1,2 one-3 sous forme d'une poudre jaune fondant à 238°C.

*Exemple 11*

Chloro-4 (chloro-3 méthyl-4 phénylamino)-5 dithiole-1,2 one-3 sous forme d'une poudre jaune fondant à 191°C.

*Exemple 12*

Chloro-4 (chloro-3 isopropyl-4 phénylamino)-5 dithiole-1,2 one-3 sous forme d'une poudre jaune fondant à 162°C.

*Exemple 13*

(Amino-4 phénylamino)-5 chloro-4 dithiole-1,2 one-3 sous forme d'une poudre jaune fondant à 195°C.

*Exemple 14*

Chloro-4 (biphénylyl-4 amino)-5 dithiole-1,2 one-3 sous forme d'une poudre jaune fondant à 167°C.

*Exemple 15*

Chloro-4 pipéridino-5 dithiole-1,2 one-3 sous forme d'une poudre marron fondant à 76°C.

*Exemple 16*

Chloro-4 morpholino-5 dithiole-1,2 one-3 sous forme d'une poudre jaune orangé fondant à 117°C.

La présente invention concerne donc les médicaments constitués par un produit de formule générale (I), sous forme libre, ou lorsqu'ils existent, sous forme de sels d'addition avec un acide pharmaceutiquement acceptables, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules de gélatine

ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprende des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement de fond de la maladie rhumatismale. Les doses dépendent de l'effet recherché et de la durée du traitement; elles sont généralement comprises entre 50 et 1000 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

L'exemple suivant illustre une composition selon l'invention.

*Exemple*

On prépare, selon la technique habituelle, des comprimés dosés à 50 mg de produit actif ayant la composition suivante:

| | |
|---|---|
| — (Amino-4 phénylamino)-5 chloro-4 dithiole-1,2 one-3 | 50 mg |
| — amidon | 60 mg |
| — lactose | 50 mg |
| — stéarate de magnésium | 2 mg |

**Revendications**

1. A titre de médicament, les dérivés de la chloro-4 dithiole-1,2 one-3 de formule générale:

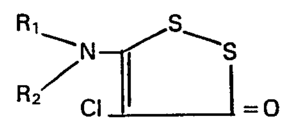

dans laquelle

— soit $R_1$ représente un atome d'hydrogène ou un radical alcoyle et $R_2$ représente un radical phényle non substitué ou substitué par un ou plusieurs substituants choisis parmi les atomes d'halogène et les radicaux alcoyle contenant 1 à 5 atomes de carbone en chaîne droite au ramifiée, hydroxy, amino, alcoylamino, dialcoylamino, alcoyloxy, alcoylthio, alcoylsulfinyle, alcanoyle, alcoyloxycarbonyle, carbamoyle, sulfamoyle, cyano, nitro, trifluorométhyle et phényle,

— soit $R_1$ et $R_2$ forment ensemble avec l'atome d'azote auquel ils sont liés un hétérocycle à 5 ou 6 chaînons pouvant contenir éventuellement un second hétéroatome choisi parmi l'oxygène et l'azote éventuellement substitué par un radical alcoyle, étant entendu que les radicaux et portions alcoyles contiennent, sauf mention spéciale, 1 à 4 atomes de carbone et sont en chaîne droite ou ramifiée, ainsi que leurs sels d'addition avec les acides.

2. Composition pharmaceutique caractérisée en ce qu'elle contient au moins un produit selon la revendication 1 en association avec un ou plusieurs diluants ou adjuvants compatibles et pharmaceutiquement acceptables.

**Patentansprüche**

1. Als Arzneimittel die Derivate des 4-Chlor-1,2--dithiol-3-ons der allgemeinen Formel

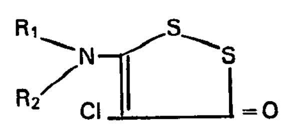

worin

— entweder $R_1$ ein Wasserstoffatom oder einen Alkylrest bedeutet und $R_2$ einen nicht-substituierten Phenylrest oder substituiert durch einen oder mehrere Substituenten, ausgewählt unter den Halogenatomen und Alkylresten mit 1 bis 5 Kohlenstoffatomen

in gerader oder verzweigter Kette, Hydroxy, Amino, Alkylamino, Dialkylamino, Alkyloxy, Alkylthio, Alkylsulfinyl, Alkanoyl, Alkyloxycarbonyl, Carbamoyl, Sulfamoyl, Cyano, Nitro, Trifluormethyl und Phenyl, bedeutet,

— oder $R_1$ und $R_2$ bilden zusammen mit dem Stickstoffatom, an das sie gebunden sind, einen Heterocyclus mit 5 oder 6 Kettengliedern, der gegebenenfalls ein zweites Heteroatom, ausgewählt aus Sauerstoff und Stickstoff, enthalten kann, gegebenenfalls substituiert durch einen Alkylrest, wobei die Alkylreste und -teile 1 bis 4 Kohlenstoffatome enthalten und in gerader oder verzweigter Kette stehen, wenn nicht anders angegeben ist, sowie ihre Additionssalze mit Säuren.

2. Pharmazeutische Zusammensetzung, dadurch gekennzeichnet, dass sie wenigstens ein Produkt gemäss Anspruch 1 zusammen mit einem oder mehreren verträglichen und pharmazeutisch annehmbaren Verdünnungs- oder Hilfsmitteln enthält.

## Claims

1. By way of a drug, the 4-chloro-1,2-dithiol-3-one derivatives of general formula:

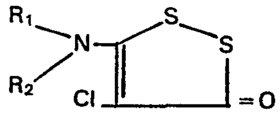

in which

— either $R_1$ denotes a hydrogen atom or an alkyl radical and $R_2$ denotes an unsubstituted phenyl radical or a phenyl radical substituted with one or more substituents chosen from halogen atoms and straight-chain or branched alkyl radicals containing 1 to 5 carbon atoms, and hydroxy, amino, alkylamino, dialkylamino, alkyloxy, alkylthio, alkylsulphinyl, alkanoyl, alkyloxycarbonyl, carbamoyl, sulphamoyl, cyano, nitro, trifluoromethyl and phenyl radicals,

— or $R_1$ and $R_2$ form, together with the nitrogen atom to which they are bound, a 5- or 6-membered heterocyclic system which can optionally contain a second heteroatom chosen from oxygen and nitrogen optionally substituted by an alkyl radical; with the proviso that, except where specifically stated, the alkyl radicals and portions contain 1 to 4 carbon atoms and are straight-chained or branched, as well as the addition salts thereof with acids.

2. Pharmaceutical composition, characterized in that it contains at least one product according to Claim 1 in combination with one or more diluents or adjuvants which are compatible and pharmaceutically acceptable.